# EUROPEAN PATENT SPECIFICATION

(11) **EP 1 868 980 B1**
(45) Date of publication and mention of the grant of the patent: **28.07.2010**
(21) Application number: 06742674.2
(22) Date of filing: 06.04.2006
(51) Int. Cl.: C07C 51/367, C07C 65/24

(54) **NEW METHOD FOR THE PREPARATION OF 6-(3-(1-ADAMANTYL)-4-METHOXPHENYL)-2-NAPHTHOIC ACID**
NEUES HERSTELLUNGSVERFAHREN FÜR 6-(3-(1-ADAMANTYL)-4-METHOXPHENYL)-2-NAPHTHOESÄURE
NOUVELLE METHODE DE PREPARATION D'ACIDE 6-Ý3-(1-ADAMANTYL)-4-METHOXYPHENYL¨-2-NAPHTOIQUE

(30) Priority: 08.04.2005 FR 0503522; 01.03.2006 US 778112 P
(43) Date of publication of application: 26.12.2007
(60) Divisional application: 10155686.8
(73) Proprietor: Galderma Research & Development, 06410 Biot (FR)
(72) Inventor: TERRANOVA, Eric Galderma Research & Development, F-06410 Biot (FR); PASCAL, Jean-Claude, Galderma Research & Development, F-06410 Biot (FR)
(74) Representative: Allab, Myriam
(86) International application number: PCT/EP2006/003790
(87) International publication number: WO 2006/108717

(56) References cited:
- EP-A- 0 199 636
- WO-A-03/011808

## Description

The present invention relates to a novel method for the manufacture of 6-[3-(1-adamantyl)-4-methoxyphenyl]-2-naphthoic acid of formula (I): 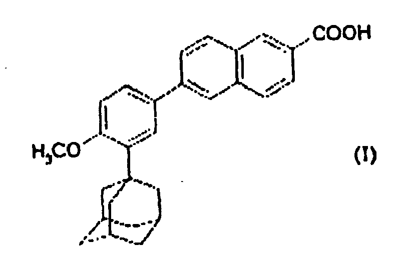 a retinoid local anti-acne agent, used in pharmaceutical compositions, in particular for the treatment of certain types of acne.

The compound of formula (I) is described in particular in patent EP 0 199 636. Patent EP 0 199 636 describes the preparation of this compound according to the following **Scheme 1:**

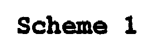

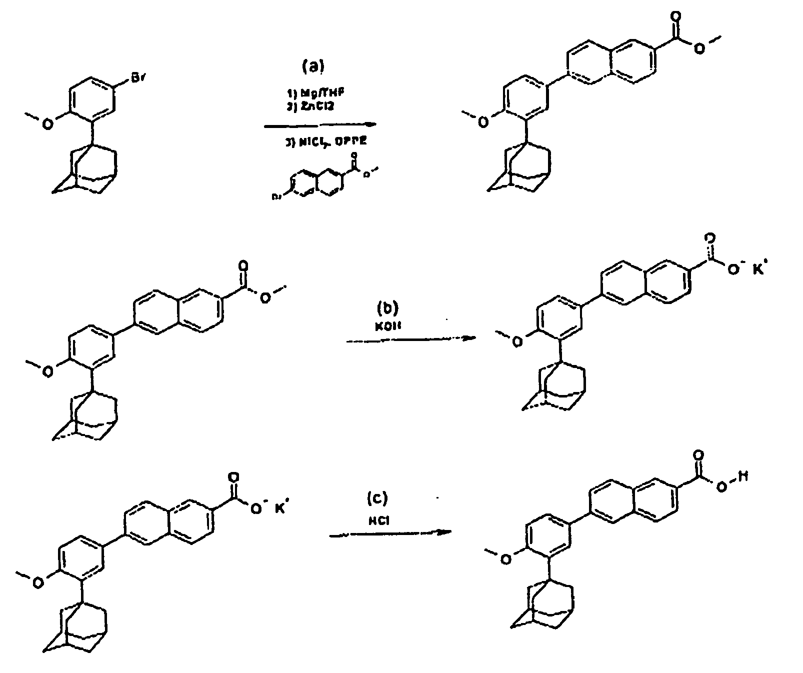

In this method, 2-(1-adamantyl)-4-bromoanisole is converted, in a first step (a), to its organomagnesium compound, and then to its organozinc compound by the action of zinc chloride (ZnCl₂), and is then coupled with methyl 6-bromonaphthoate. This reaction is catalyzed by a transition metal (palladium or nickel) or one of its complexes with various phosphines. The synthesis of 6-[3-(1-adamantyl)-4-methoxyphenyl]-2-naphthoic acid of formula (I), as described in patent EP 0 199 636 (Scheme 1), is therefore carried out in three steps with a yield of 63%, from 2-adamantyl-4-bromoanisole.

One of the disadvantages of this reaction is the formation of impurities. One of these impurities results from the reaction of the organozinc compound generated *"in situ"* with 2-adamantyl-4-bromoanisole to give 3,3'-di(1-adamantyl)-4,4'-dimethoxy-1,1'-biphenyl having the following structure:

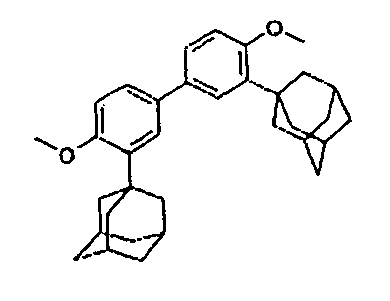

Another impurity also forms via the transfer of the zinc compound of 2-(1-adamantyl)-4-bromoanisole to methyl 6-bromonaphthoate and the reaction of this novel zinc compound with methyl 6-bromonaphthoate to give the dimerization product having the structure:

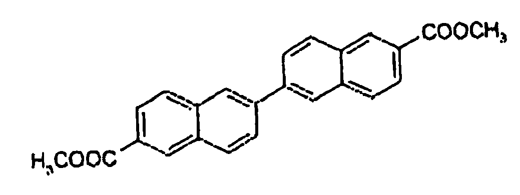

This product leads, after saponification (step (b)) and acidification (step (c)), to the following impurity:

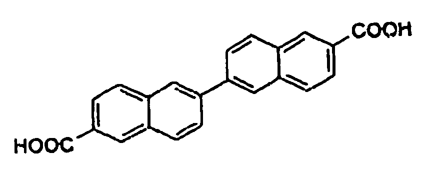

A third impurity forms during the hydrolysis of the reaction medium. Indeed, during this hydrolysis, the unreacted organozinc compound of 2-(1-adamantyl)-4-bromoanisole generates the impurity having the following structure:

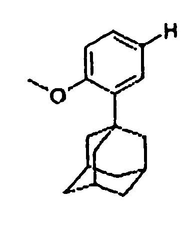

At an industrial stage, these impurities are difficult to remove from the finished product and most often require reprocessing by recrystallization.

Furthermore, some catalysts such as [1,2-bis(diphenylphosphino)ethane]nickel chloride (NiCl₂(dppe)) must be prepared separately, adding a step to this method.

During the coupling reaction in step (a), the acid functional group is protected in methyl ester form. This acid functional group should be regenerated. Thus, in a second step (b), methyl 6-[3-(1-adamantyl)-4-methoxyphenyl]-2-naphthoate is saponified by treating with a base such as sodium hydroxide (NaOH) or potassium hydroxide (KOH) under reflux in an alcohol such as methanol.

By acidifying the reaction medium with hydrochloric acid, 6-[3-(1-adamantyl)-4-methoxyphenyl]-2-naphthoic acid is obtained in a third step (c). It should also be noted that in this method, the methyl ester of 6-bromo-naphthoic acid should be prepared in one step from the corresponding acid. It can therefore be seen that the prior art method is complex and is not entirely satisfactory.

In this context, one of the objects of the present invention is to provide a method for preparing 6-[3-(1-adamantyl)-4-methoxyphenyl]-2-naphthoic acid, which is a lot simpler and more economical. The method according to the invention should be more suited to an industrial production, more particularly in terms of cost and compliance with Good Manufacturing Practices. One of the objectives of the invention is to provide a novel method for preparing compound (I) which does not require in the final stage deprotection of the acid functional group, avoiding the formation of the impurities mentioned above and making it possible to reduce the number of synthesis steps.

In this context, the subject of the present invention is a method for preparing 6-[3-(1-adamantyl)-4-methoxyphenyl]-2-naphthoic acid of formula (I): 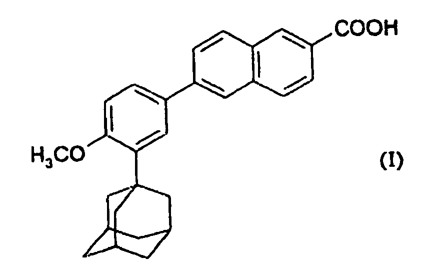 by a single-step Suzuki reaction between 3-adamantyl-4-methoxyphenylboronic acid of formula (II): 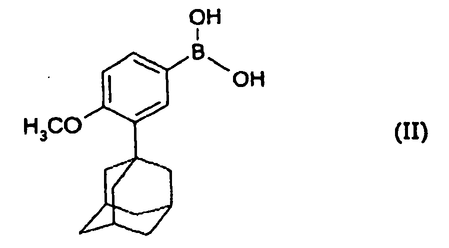 and 6-bromo-2-naphthoic acid of formula (III):

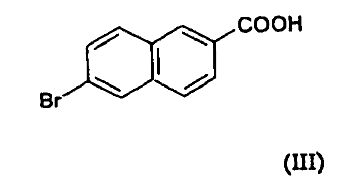

The subject of the invention is also the use of the compound (II), on the one hand, and the use of the compound (III), on the other hand, for the preparation of the compound of formula (I).

According to the method of the invention, it is therefore possible to couple in a single step the (1-adamantyl)phenyl part and the naphthyl part of 6-[3-(1-adamantyl)-4-methoxyphenyl]-2-naphthoic acid.

The preparation of 6-[3-(1-adamantyl)-4-methoxyphenyl]-2-naphthoic acid, according to the method of the invention, is illustrated in **Scheme 2** below:

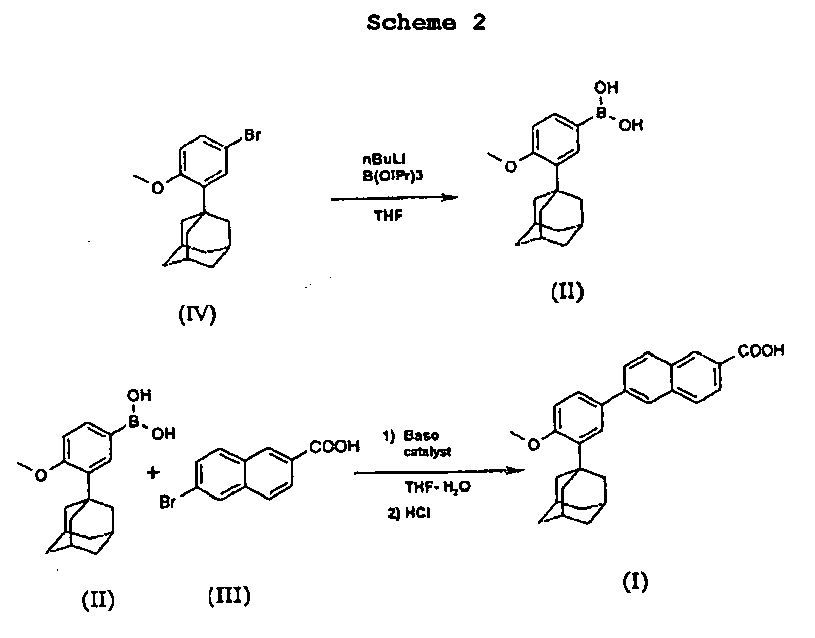

The preparation of 6-[3-(1-adamantyl)-4-methoxyphenyl]-2-naphthoic acid (I) is carried out by the Suzuki reaction between 3-adamantyl-4-methoxyphenylboronic acid (II) (prepared in particular according to a method similar to that described in patent applications WO 02/072009 A2 and WO 03/011808 A1) and the commercially available 6-bromo-2-naphthoic acid (III). The preparation of 6-[3-(1-adamantyl)-4-methoxyphenyl]-2-naphthoic acid of formula (I) as described by this novel method according to Scheme 2 is carried out in two steps from 2-(1-adamantyl)-4-bromcanisole (IV) (prepared for example according to EP 0 199 636), with a yield which is much higher than that obtained with the prior art method. As shown by the example which follows, the yield of the method according to the invention may be of the order of 95% or higher.

In the context of the invention, the Suzuki reaction is carried out by coupling between compounds (II) and (III) in the presence of a palladium catalyst and a base, in a polar solvent, followed by an acid treatment.

Typically, the Suzuki reaction may be carried out in the presence of a palladium catalyst such as palladium(II) acetate, tetrakis(triphenylphosphine)-palladium(0), palladium on activated charcoal or dichloro[1,1'-bis(diphenylphosphino)ferrocene]-palladium(II), in an aprotic polar solvent (for example acetonitrile, N,N-dimethylformamide, dimethoxyethane or tetrahydrofuran) or a protic polar solvent (for example n-propanol, i-propanol) or a mixture of these solvents with water. The volume of solvent used will be between 7 and 13 times the quantity of 6-bromo-2-naphthoic acid (III) used and the volume of water used will be between 7 and 13 times the quantity of 6-bromo-2-naphthoic acid (III) used.

Advantageously, the palladium catalyst may contain a ligand chosen from: a triphenylphosphine, a tri-o-tolylphosphine, a tri-m-tolylphosphine or a tri-p-tolylphosphine. The catalysts particularly preferred are palladium(II) acetate and palladium on carbon which make it possible to obtain a particularly fast reaction kinetics. Palladium(II) acetate may be advantageously used in combination with a 2-(dicyclohexylphosphino)-biphenyl type ligand (J.P. Wolfe et al., J. Am. Chem. Soc., 1999, 121, 9550-9561).

These catalysts may also be encapsulated, like for example the Pd EnCat^{™} type catalysts. The reaction is generally carried out in the presence of an inorganic base such as potassium carbonate, sodium carbonate, caesium carbonate, sodium hydroxide or potassium hydroxide or in the presence of a tertiary amine such as triethylamine or diisopropylethylamine. The particularly preferred bases are potassium carbonate, potassium hydroxide and diisopropylethylamine.

The Suzuki reaction is preferably carried out under an inert atmosphere, for example under an argon or nitrogen atmosphere. The reaction mixture is advantageously heated at a temperature in the range from 60° to 110°C, for 30 minutes to 24 hours. A treatment in an acidic medium, for example in the presence of HCl, is carried out. It will be noted that, according to the conditions used in Examples 1 and 2, the kinetics of the reaction is very rapid and is complete within two hours. Persons skilled in the art will be able to modify these conditions, in particular by applying the variants of the Suzuki reaction which are described in the literature (N. Miyaura & A. Suzuki, Chem. Rev., 1995, 95, 2457-2483; A. Suzuki, J. Organomet. Chem., 1999, 576, 147-168). The method according to the invention is therefore simple and economical and makes it possible to directly obtain compound (I) with a high yield, close to quantitative.

This new method also makes it possible to obtain 6-[3-(1-adamantyl)-4-methoxyphenyl]-2-naphthoic acid with a high degree of purity in which the impurities obtained in the prior art method are completely absent. The following preparations illustrate the method of the invention.

### Example 1:

### a) - Preparation of 3-adamantyl-4-methoxyphenylboronic acid (II):

100 g (0.311 mol) of 2-(1-adamantyl)-4-bromoanisole (IV) and 500 ml of THF are introduced, under nitrogen, into a 2 L three-necked reactor. The reaction medium is cooled to -75°C. 137 ml (0.342 mol) of a 2.5 M nBuLi solution are added. After stirring for 1 h at -70°C, 80 ml (0.342 mol) of triisopropyl borate are added. After returning to room temperature, the reaction mixture is hydrolysed with 1 litre of 1.2 N HC1. The aqueous phase is extracted with ethyl acetate and the combined organic phases are washed with 1 litre of saturated NaCl, and then with 1 litre of water. The organic phases are dried over sodium sulphate and the solvents are evaporated. 88.37 g of a white solid are obtained, which solid is reimpasted in 440 mL of heptane. After filtration, the precipitate obtained is rinsed with heptane, and then dried under reduced pressure at 35°C until a constant weight is obtained. 84.4 g of 3-adamantyl-4-methoxyphenylboronic acid are obtained in the form of a white solid - (yield = 94.8%; m.p. = 263°C).

**¹H NMR (CDCl₃):** δ: 1.77 (s; 6H); 2.10 (m; 3H); 2.20 (s; 6H); 3.91 (s; 3H); 7.00 (d; 1H; J₁=8.0 Hz); 8.05 (dxd; 1H; J₂=1.5 Hz and J₁=8.0 Hz); 8.15 (d; 1H, J₂=1.5 Hz)

### b) - Preparation of 6-[3-(1-adamantyl)-4-methoxyphenyl]-2-naphthoic acid (I):

20 mL of tetrahydrofuran (12 vol), 2 g (7 mmol) of 3-adamantyl-4-methoxyphenylboronic acid (II), 1.65 g (6.6 mmol) of 6-bromo-2-naphthoic acid (III) and 20 mL of a 2 M aqueous potassium carbonate solution are introduced into a round-bottomed flask equipped with stirring and under a nitrogen stream. 15 mg (1%) of palladium acetate and 46 mg (2%) of 2-(dicyclohexyl-phosphino)biphenyl are then introduced. The medium is heated under reflux for 2 hours. Kinetic monitoring by HPLC indicates that the % of 6-[3-(1-adamantyl)-4-methoxyphenyl]-2-naphthoic acid formed is 94% after one hour and 98% after 2 h.

After returning to room temperature, the catalyst is filtered on a cartridge, and then slowly poured over 30 ml of a 1 N aqueous hydrochloric acid solution.

The medium is kept stirring for one hour. The precipitate is filtered, washed with water and then dried under reduced pressure. 2.68 g of 6-[3-(1-adamantyl)-4-methoxyphenyl]-2-naphthoic acid are obtained in the form of a white solid whose purity, determined by HPLC, is 99.9% (yield = 94.8%; m.p. = 321°C) .

The following melting points (m.p.) exist in the literature: m.p. = 319°-322°C (B. Charpentier et al., J. Med. Chem., 1995, 38, 4993-5006) and m.p. = 325°-327°C (EP 0 199 636).

### Example 2:

### Preparation of 6-[3-(1-adamantyl)-4-methoxyphenyl]-2-naphthoic acid (I):

80 g (0.319 mol) of 6-bromo-2-naphthoic acid, 95.7 g (0.335 mol, 1.05 eq) of 3-adamantyl-4-methoxyphenyl-boronic acid, 0.8 g of 5% palladium on carbon (50% wet, Degussa type E105CA/W) and 800 ml of tetrahydrofuran (10 vol) are introduced into a 4 litre reactor. The medium is heated to 55°C. 85 g (1.05 mol, 3.3 eq) of potassium hydroxide at 85% are dissolved in 240 ml of water (3 vol).

The solution obtained is poured over the reaction medium. The addition is exothermic. The reaction medium reaches the reflux temperature. The reflux is maintained for about 2 hours.

The reaction medium is filtered at about 35-40°C on a cartridge and rinsed with 400 ml of a THF/water mixture (1/1).

The medium is cooled to 20°C and 100 ml of HCl at 35% in 600 ml of water are added. 6-[3-(1-adamantyl)-4-methoxyphenyl]-2-naphthoic acid precipitates. It is filtered and washed with 4 litres of water. The pH of the washings is about 6-7. The product is dried under vacuum at 100°C for 24 hours.

131 g of 6-[3-(1-adamantyl)-4-methoxyphenyl]-2-naphthoic acid are obtained (crude yield = 99%).

This crude material is dissolved in 15 to 22 volumes of THF under reflux. After filtration in the hot state, 15 to 22 volumes of heptane are added and the medium is cooled to about 5°C for 1 to 2 hours.

The 6-[3-(1-adamantyl)-4-methoxyphenyl]-2-naphthoic acid is filtered on sintered glass and it is rinsed with 1 to 2 volumes of heptane.

108 g of 6-[3-(1-adamantyl)-4-methoxyphenyl]-2-naphthoic acid are obtained in the form of a white solid whose purity, determined by HPLC, is 99.9% (yield = 82%; m.p. = 320-322°C).

### Example 3:

### Preparation of 6-[3-(1-adamantyl)-9-methoxyphenyl]-2-naphthoic acid (I):

20 ml (12 vol) of tetrahydrofuran, 2 g (7 mmol) of 3-adamantyl-4-methoxyphenylboronic acid (II), 1.65 g (6.6 mmol) of 6-bromo-2-naphthoic acid (III) and 20 mL of a 2 M aqueous potassium carbonate solution are introduced into a round-bottomed flask equipped with stirring and under a nitrogen stream. 0.7 g (5%) of 10% palladium on carbon (50% wet; Heraeus type K-0218) is then introduced.

The medium is heated under reflux for 8 hours. The catalyst is filtered on a cartridge, and then slowly poured over 30 ml of a 1 N aqueous hydrochloric acid solution.

The medium is kept stirring for one hour. The precipitate is filtered, washed with water and then dried under reduced pressure. 2.06 g of 6-[3-(1-adamantyl)-4-methoxyphenyl]-2-naphthoic acid are obtained in the form of a white solid whose purity, determined by HPLC, is 99.9% (yield = 79%; m.p. = 321°C).

## Claims

1. Method for preparing 6-[3-(1-adamantyl)-4-methoxyphenyl]-2-naphthoic acid of formula (I): 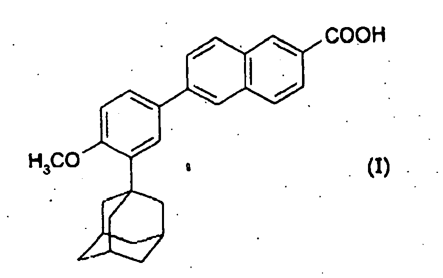 by a Suzuki reaction between 3-adamantyl-4-methoxyphenylboronic acid of formula (II): 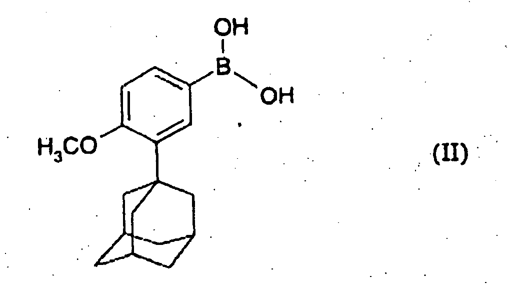 and 6-bromo-2-naphthoic acid of formula (III):

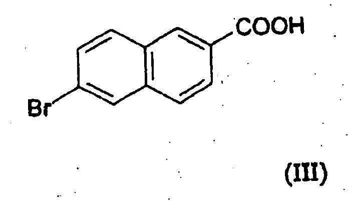

2. Method according to Claim 1, **characterized in that** the Suzuki reaction is carried out by coupling between compounds (II) and (III) in the presence of a palladium catalyst and a base, in a polar solvent, followed by an acid treatment.

3. Method according to Claim 2, **characterized in that** the catalyst is chosen from palladium(II) acetate, palladium on activated charcoal, dichloro[1,1'-bis(diphenylphosphino)ferrocene]palladium(II), and the palladium complexes containing a phosphine ligand, such as tetrakis(triphenylphosphine)palladium.

4. Method according to Claim 3, **characterized in that** the phosphine ligand is chosen from 2-(dicyclohexylphosphino)biphenyl, triphenylphosphine, tri-o-tolylphosphine, tri-m-tolylphosphine or tri-p-tolylphosphine.

5. Method according to Claim 3, **characterized in that** the catalyst is palladium(II) acetate or palladium on activated charcoal.

6. Method according to Claim 5, **characterized in that** the catalyst is palladium(II) acetate in the presence of the ligand 2-(dicyclohexylphosphino)biphenyl.

7. Method according to one of Claims 2 to 6, **characterized in that** the baste is an inorganic base chosen from potassium carbonate, sodium carbonate, caesium carbonate, sodium hydroxide or potassium hydroxide, or a tertiary amine chosen from triethylamine and diisopropylethylamine.

8. Method according to one of Claims 2 to 7, **characterized in that** the polar solvent is chosen from acetonitrile, N,N-dimethylformamide, dimethoxyethane, tetrahydrofuran, n-propanol, i-propanol or a mixture of these solvents with water.

9. Method according to one of Claims 2 to 8, **characterized in that** the coupling is carried out at a temperature in the range from 60 to 110°C, for 30 minutes to 24 hours, under an inert argon or nitrogen atmosphere.

10. Method according to one of Claims 2 to 9, **characterized in that** the acid treatment is carried out with hydrochloric acid.

11. Use of 3-adamantyl-4-methoxyphenylboronic acid of formula (II) 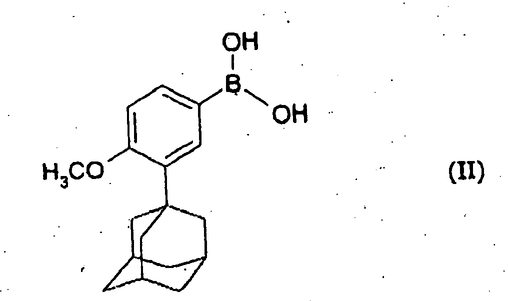 for the preparation of 6-[3-'(1-adamantyl)-4-methoxyphenyl]-2=naphthoic acid of formula (I):

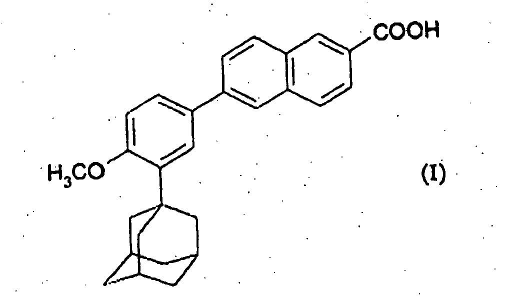

12. Use of 6-bromo-2-naphthoic acid of formula (III): 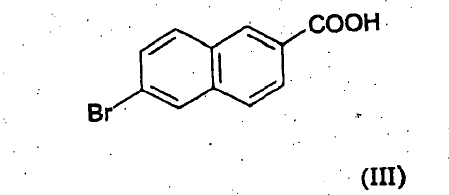 for the preparation of 6-[3-(1-adamantyl)-4-methoxyphenyll-2-naphthoic acid of formula (I): 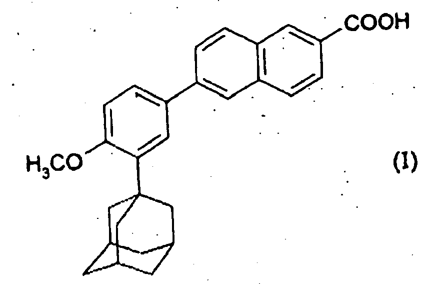 by a Suzuki coupling reaction.

13. Method according to one of Claims 1 to 8, **characterized in that** the volume of solvent used is between 7 and 13 times the quantity of 6-bromo-2-naphthoic acid (III) used and the volume of water used is between 7 and 13 times the quantity of 6-bromo-2-naphthoic acid (III) used.

## Patentansprüche

1. Verfahren zur Herstellung der 6-[3-(1-Adamantyl)-4-methoxyphenyl]-2-naphthoesäure der Formel (I): 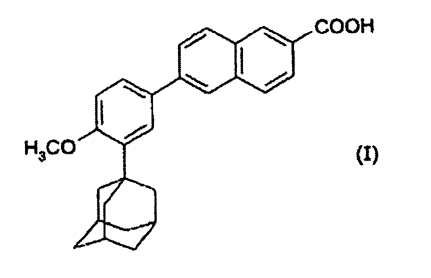 durch eine Suzuki-Reaktion der 3-Adamantyl-4-methoxyphenylboronsäure der Formel (II): 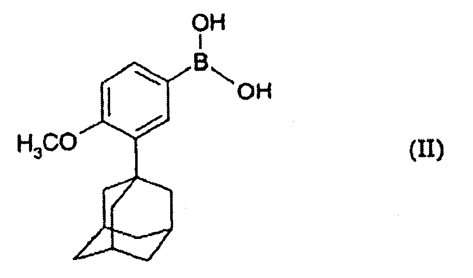 und der 6-Brom-2-naphthoesäure der Formel (III):

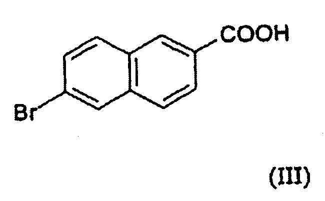

2. Verfahren nach Anspruch 1, **dadurch gekennzeichnet, dass** die Suzuki-Reaktion durchgeführt wird, indem die Verbindungen (II) und (III) in Gegenwart eines Palladiumkatalysators und einer Base in einem polaren Lösemittel gekuppelt werden und anschließend mit einer Säure behandelt wird.

3. Verfahren nach Anspruch 2, **dadurch gekennzeichnet, dass** er Katalysator unter Palladium(II)acetat, Palladium auf Aktivkohle, Dichlor-[1,1'-bis(diphenylphosphino)ferrocen]-palladium(II), und Palladiumkomplexen ausgewählt ist, die einen Phosphinliganden aufweisen, wie Tetrakis(triphenylphosphin)palladium.

4. Verfahren nach Anspruch 3, **dadurch gekennzeichnet, dass** der Phosphinligand unter 2-(Dicyclohexyl-phosphino)biphenyl, Triphenylphosphin, Tri-*o*-tolyl-phosphin, Tri-m-tolylphosphin oder Tri-*p*-tolylphosphin ausgewählt ist.

5. Verfahren nach Anspruch 3, **dadurch gekennzeichnet, dass** es sich bei dem Katalysator um das Palladium(II)acetat oder Palladium auf Aktivkohle handelt.

6. Verfahren nach Anspruch 5, **dadurch gekennzeichnet, dass** es sich bei dem Katalysator um Palladium(II)acetat in Gegenwart des Liganden 2-(Dicyclohexyl-phosphino)biphenyl handelt.

7. Verfahren nach einem der Ansprüche 2 bis 6, **dadurch gekennzeichnet, dass** die Base eine anorganische Base ist, die unter Kaliumcarbonat, Natriumcarbonat, Cäsiumcarbonat, Natriumhydroxid oder Kaliumhydroxid ausgewählt ist, oder ein tertiäres Amin, das unter Triethylamin und Düsopropylethylamin ausgewählt ist.

8. Verfahren nach einem der Ansprüche 2 bis 7, **dadurch gekennzeichnet, dass** das polare Lösemittel unter Acetonitril, N,N-Dimethylformamid, Dimethoxyethan, Tetrahydrofuran, n-Propanol, Isopropanol oder einem Gemisch dieser Lösemittel mit Wasser ausgewählt ist.

9. Verfahren nach einem der Ansprüche 2 bis 8, **dadurch gekennzeichnet, dass** die Kupplung bei einer Temperatur im Bereich von 60 bis 110 °C während 30 Minuten bis 24 Stunden unter einer inerten Argonatmosphäre oder Stickstoffatmosphäre durchgeführt wird.

10. Verfahren nach einem der Ansprüche 2 bis 9, **dadurch gekennzeichnet, dass** die Säurebehandlung mit Salzsäure durchgeführt wird.

11. Verwendung der 3-Adamantyl-4-methoxyphenylboronsäure der Formel (II): 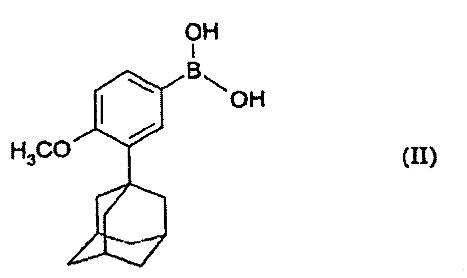 für die Herstellung der 6-[3-(1-Adamantyl)-4-methoxyphenyl]-2-naphthoesäure der Formel (I):

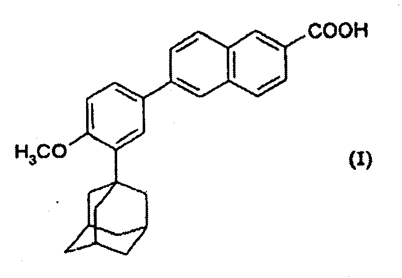

12. Verwendung der 6-Brom-2-naphthoesäure der Formel (III): 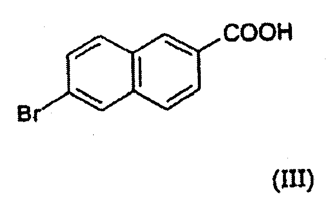 für die Herstellung der 6-[3-(1-Adamantyl)-4-methoxyphenyl]-2-naphthoesäure der Formel (I): 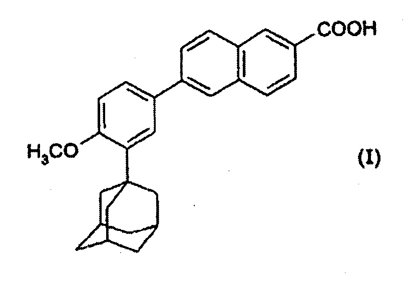 gemäß einer Suzuki-Kupplung.

13. Verfahren nach einem der Ansprüche 1 bis 8, **dadurch gekennzeichnet, dass** das verwendete Lösemittelvolumen das 7-bis 13-fache der verwendeten Menge der 6-Brom-2-naphthoesäure (III) und das verwendete Wasservolumen das 7-bis 13-fache der verwendeten Menge der 6-Brom-2-naphthoesäure (III) beträgt.

## Revendications

1. Procédé de préparation de l'acide 6-[3-(1-adamantyl)-4-méthoxyphényl]-2-naphtoïque de formule (I) : 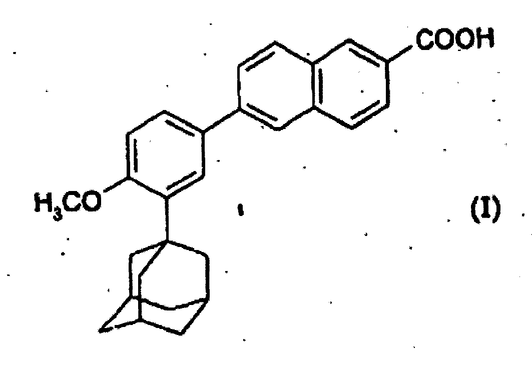 par une réaction de Suzuki entre l'acide 3-adamantyl-4-méthoxyphénylborique de formule (II) : 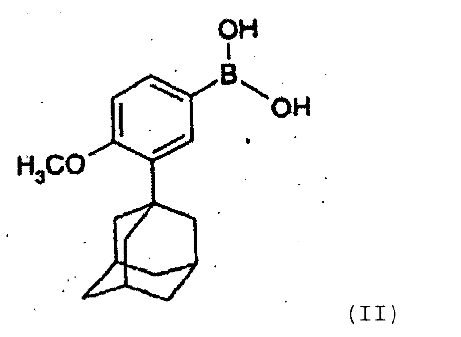 et l'acide 6-bromo-2-naphtoïque de formule (III) :

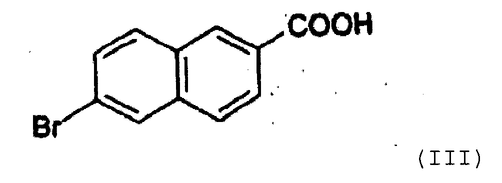

2. Procédé selon la revendication 1, **caractérisé en ce que** la réaction de Suzuki est conduite par couplage entre les composés (II) et (III) en présence d'un catalyseur au palladium et d'une base, dans un solvant polaire, suivi par un traitement acide.

3. Procédé selon la revendication 2, **caractérisé en ce que** le catalyseur est choisi parmi l'acétate de palladium (II), le palladium sur charbon actif, le dichloro[1,1'-bis(diphénylphosphino)ferrocène]-palladium (II), et les complexes de palladium contenant un ligand phosphine, tel que le tétrakis(triphényl-, phosphine)palladium.

4. Procédé selon la revendication 3, **caractérisé en ce que** le ligand phosphine est choisi parmi le 2-(dicyclohexylphosphino)biphényle, la triphénylphosphine, la tri-o-tolylphosphine, la tri-m-tolylphosphine ou la tri-p-tolylphosphine.

5. Procédé selon la revendication 3, **caractérisé en ce que** le catalyseur est l'acétate de palladium (II) ou le palladium sur charbon actif.

6. Procédé selon la revendication 5, **caractérisé en ce que** le catalyseur est l'acétate de palladium (II) en présence du ligand 2-(dicyclohexylphosphino)-biphényle.

7. Procédé selon une des revendications 2 à 6, **caractérisé en ce que** la base est une base inorganique choisie parmi le carbonate de potassium, le carbonate de sodium, le carbonate de césium, l'hydroxyde de sodium ou l'hydroxyde de potassium, ou une amine tertiaire choisie par la triéthylamine et la diisopropyléthylamine.

8. Procédé selon l'une des revendications 2 à 7, **caractérisé en ce que** le solvant polaire est choisi parmi l'acétonitrile, le N,N-diméthylformamide, le diméthoxyéthane, le tétrahydrofurane, le n-propanol, l'i-propanol ou un mélange de ces solvants avec de l'eau.

9. Procédé selon l'une des revendications 2 à 8, **caractérisé en ce que** le couplage est conduit à une température dans la plage de 60 à 110°C, pendant 30 minutes à 24 heures, sous une atmosphère inerte d'argon ou d'azote.

10. Procédé selon l'une des revendications 2 à 9, **caractérisé en ce que** le traitement acide est conduit avec de l'acide chlorhydrique.

11. Utilisation de l'acide 3-adamantyl-4-méthoxyphénylborique de formule (II) 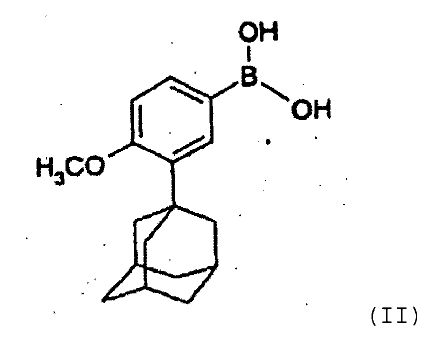 pour la préparation de l'acide 6-[3-(1-adamantyl)-4-méthoxyphényl]-2-naphtoïque de formule (I) :

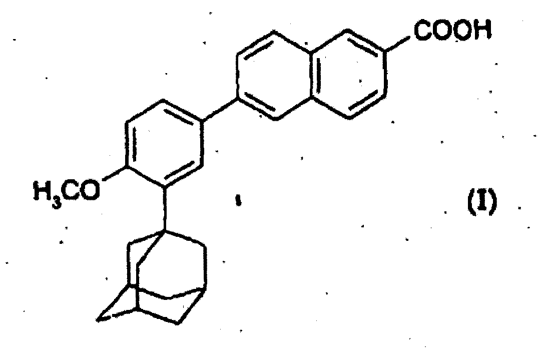

12. Utilisation de l'acide 6-bromo-2-naphtoïque de formule (III) : 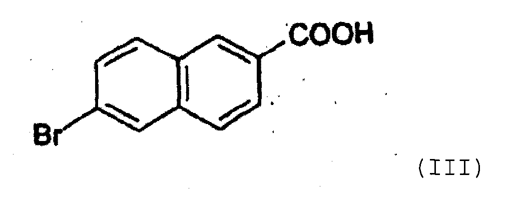 pour la préparation de l'acide 6-[3-(1-adamantyl)-4-méthoxyphényl]-2-naphtoïque de formule (I) : 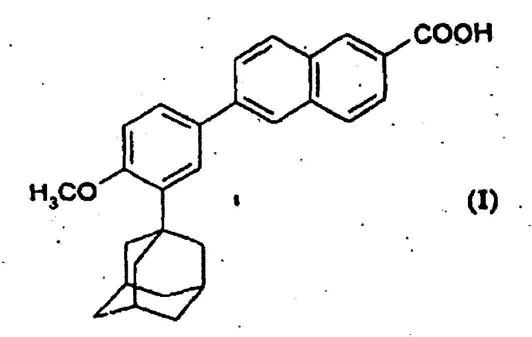 par une réaction de couplage de Suzuki.

13. Procédé selon l'une des revendications 1 à 8, **caractérisé en ce que** le volume de solvant utilisé est entre 7 et 13 fois la quantité d'acide 6-bromo-2-naphtoïque (III) utilisée et le volume d'eau utilisé est entre 7 et 13 fois la quantité d'acide 6-bromo-2-naphtoïque (III) utilisée.
